(19) Europäisches Patentamt
European Patent Office
Office européen des brevets

(11) Publication number: 0 385 106 B1

(12) # EUROPEAN PATENT SPECIFICATION

(45) Date of publication of patent specification: **23.03.94**

(51) Int. Cl.5: **A61K 9/22**, A61K 31/71

(21) Application number: **90101483.7**

(22) Date of filing: **25.01.90**

(54) **Sustained release bolus effective for the prolonged prevention, treatment or control of nematode, acarid and endo- and ectoparasitic infestations of ruminants.**

(30) Priority: **28.02.89 US 316625**

(43) Date of publication of application:
**05.09.90 Bulletin 90/36**

(45) Publication of the grant of the patent:
**23.03.94 Bulletin 94/12**

(84) Designated Contracting States:
**AT BE CH DE DK ES FR GB GR IT LI LU NL SE**

(56) References cited:
**EP-A- 0 094 779**
**EP-A- 0 236 901**

**B.W.BYCROFT "Dictionary of Antibiotics and Related Substances" 1988 CHAPMAN AND HALL LTD., London page 91**

(73) Proprietor: **AMERICAN CYANAMID COMPANY**
**One Cyanamid Plaza**
**Wayne, NJ 07470-8426(US)**

(72) Inventor: **Wood, Irwin Boyden**
**211 Elm Avenue**
**Yardley, Pennsylvania 19067(US)**
Inventor: **Toothill, Richard Boardman**
**16 Sunrise Drive**
**Warren, New Jersey 07060(US)**
Inventor: **Dietz, Joseph Charles**
**7282 Prince Drive**
**Terre Haute, Indiana 47802(US)**

(74) Representative: **Wächtershäuser, Günter, Dr.**
**Tal 29**
**D-80331 München (DE)**

EP 0 385 106 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to bolus compositions containing a prophylactically effective amount of the compound LL-F28249α, 23-(O-methyloxime)LL-F28249α or derivative thereof and the administration thereof to ruminant animals to prevent or control nematode, endoparasitic insect, ectoparasitic insect or acarid infestations in said animals for a prolonged period of time.

More particularly, the present invention relates to novel sustained release boluses comprising about 0.3% to 10.0% by weight of LL-F28249α, 23-(O-methyloxime)LL-F28249α or derivative thereof, about 10.0% to 20.0% by weight of glycerol monostearate, about 3.0% to 10.0% by weight of carnauba wax and about 70.0% to 85.0% by weight of barium sulfate. The boluses of the invention are effective for protecting ruminant animals for a prolonged period of time against infestation by nematodes, endo- and ectoparasitic insects and acarids, and for decontaminating pastures to eliminate the infective stages of said parasites by orally administering to said ruminants a bolus, as described above, which continuously releases into the rumen of the treated animals, for a prolonged period of time, a therapeutically or prophylactically effective amount of the LL-F28249α, 23-(O-methyloxime)LL-F28249α or a derivative thereof.

Surprisingly, it is also found that control or prevention of nematode infestations in ruminants can be achieved with extremely low levels of LL-F28249α or 23-(O-methyloxime)LL-F28249α administered in a bolus of the invention comprising about 0.3% to less than 1.0% by weight of LL-F28249α, 23-(O-methyloxime)LL-F28249α or derivative thereof; about 14% to 16% by weight glycerol monostearate; about 3% to 5% by weight carnauba wax and about 78.01% to 82.7% by weight of barite.

Moreover, we have found that the boluses of the present invention are effective for providing prophylactic control of parasites that affect livestock and that such control is apparently derived from the continuous presence of exceedingly low levels of LL-F28249α or 23-(O-methyloxime)LL-F28249α. It is also observed that this prophylactic treatment of ruminants not only renders the animals free of infestation from nematodes, endoparasitic insects, ectoparasitic insects and acarids, but in addition provides significantly improved weight gains over untreated animals or animals treated with other anthelmintic, antibacterial or acaricidal agents.

## DESCRIPTION OF THE PREFERRED EMBODIMENTS

Preferred bolus compositions effective for control and/or prevention of insect, nematode and acarid infestations of ruminants such as cattle, sheep, goats, bison, buffalo, deer and the like, comprise about 1.0 to 8.5% by weight of LL-F28249α or 23-(O-methyloxime)LL-F28249α, about 14.0% to 16.0% by weight of glycerol monostearate, about 3.0% to 5.0% by weight of carnauba wax and about 70.0% to 80.5% of barite.

One preferred embodiment of the present invention is a sustained release bolus for oral administration to ruminant animals, effective for protecting said animals for a prolonged period (up to about 6 months) against adult and larval infestation by helminths, endoparasitic insects ectoparasitic insects and acarids, consisting essentially of about 0.3% to 10% by weight of LL-F28249α, 23-(O-methyloxime-LL-F28249α or derivative thereof; about 10% to 20% by weight ofglycerol monostearate; about 3.0% to 10% by weight of carnauba wax and about 70.0% to 85% by weight of barium sulfate or barite fines. One especially preferred sustained release bolus consists essentially of about 0.3% to less than 1% by weight of LL-F28249α or 23-(o-methyloxime)LL-F28249α, about 14% to 16% by weight of glycerol monostearate, about 3.0% to 5.0% by weight of carnauba wax and about 78.01% to 82.7% by weight of barium sulfate or barite fines. Other preferred embodiments of the present invention are as defined in claims 4 to 10.

Surprisingly the prophylactic treatment of ruminants (exceedingly low dosage per day in some embodiments) not only prevents and/or controls nematodes, endo- and ectoparasitic insects and acarids, but also provides significantly improved weight gains over untreated animals or animals treated with other anthelmintic, antibacterial or acaricidal agents.

The preparation of sustained release bolus formulations of this invention is accomplished by blending on a weight basis, at a temperature below the melting point of the wax or mixture, about 0.3% to 10% active ingredient; about 3.0% to 10% carnauba wax; about 10% to 20% of glycerol monostearate and about 70% to 85% of a high-density, pharmacologically and pharmaceutically acceptable filler selected from barium sulfate and barite. The blend may be used in the injection molding step as is, or the blend may be pelletized or flaked using an extruder or flaker and the resulting pellets or flakes used for injection molding.

After blending, the mixture is discharged and either transferred to a pelletizing extruder or directly charged into the injection molder. If pelletizing is desired for improved homogeneity, the mixture may be pelletized utilizing a three-stage temperature profile comprising a feed section maintained at a temperature below the melting point of the wax, a hot transition section maintained at a temperature above the melting

point of the wax and stearate, and a metering section maintained at a temperature below the melting point of the wax and stearate employing with a straight-through die equipped with a breaker plate, the die equipped breaker plate assembly being maintained at a temperature range which is similar to the range of the metering section.

The resulting extruded material is air cooled and cut into pellets of the desired size. Improved homogeneity may also be obtained by flaking the blend of active ingredient, wax, and barium sulfate or barite filler, and then prilling or wax granulating with commercially available equipment to produce better homogeneity.

The resulting pellets and/or dry blend materials are transferred to an injection molding system which is operated with a three stage temperature profile similar to that employed for pelletization; i.e, a feed phase below the mixture softening point, a transition phase above the softening point, and an injection phase wherein the mixture is injected into a mold at a temperature below the softening point of the blend. The mold is cooled to solidify the bolus. Boluses produced are then removed from the mold and packaged.

While the above process may be carried out with barium sulfates or other fillers and weighting agents, barite fines are preferred. The use of barite fines in the process has several advantages over the use of purified USP grade barium sulfate, for example the use of barite increases the hardness of the bolus, and reduces the erosion rate of the bolus. Thus, the bolus provides a more controlled erosion rate and more sustained release rate of the LL-F28249α or the 23-(O-methyloxime)LL-F28249α.

Also, during the processing of the mixtures containing the wax, glycerol monostearate and active ingredient, it is found that the use of barite fines rather than USP grade barium sulfate produces less tacky and more easily handled solid mixtures.

Preparation of the boluses of this invention by injection molding avoids the need for cryogenic grinding of the solid mixture or the use of additional lubricants frequently required in other methods, such as tablet pressing of solids. Another advantage is that recycling of particles retained on the (100 mesh) screens with 0.149 mm openings is avoided. Tablet pressing requires (100 mesh) material passing through screens with 0.149 mm openings for pressing since use of a (>100 mesh) material not passing through screens with 0.149 mm openings results in faster release of active agents from the bolus.

In a preferred method for the preparation of the LL-F28249α and 23-(0-methyloxime)LL-F28249α boluses of this invention by injection molding the cycle time is reduced to about 40 to 50 seconds. Full potency can be expected when operating conditions are maintained below 140 °C. Actual operating conditions which appear to be highly desirable include: a) increasing temperature profile, i.e., an ambient barrel and a 90 °C nozzle temperature; b) injection time 20 sec.; c) injection pressure 70.3 $kg/cm^2$ or 68.95 Bars (0.5 tons psig) and d) a cooling time of 20 sec. These conditions result in a melt temperature between 89 to 90 °C, well below the critical temperature for decomposition. Time/temperature studies have indicated that continual recycle of sprues and runners is possible due to this excellent stability. The injection molded boluses have a higher density, are harder and seem to erode at a slower rate (as determined by methanol disintegration) than poured boluses. Also, rheology data indicate that the better "wettability" of barite may protect the active ingredient from thermal decomposition.

While injection molding is a preferred method for preparation of boluses of this invention, it should be recognized that said boluses can be prepared by hand molding techniques. It should also be recognized that the size of the boluses can be significantly altered to provide bolus sizes for all sizes and species of ruminant animals. Using a hand molding technique, the monostearate and carnauba wax are blended, melted and heated to 105± 5 deg. C. To this melt is added the active ingredient and blended to a uniform suspension. To this suspension is added the barite with continual mixing. Blending continues until a uniform creamy mixture is obtained. The resulting material is poured into a mold, allowed to cool to room temperature and demolded. The size and weight of the boluses prepared are, of course, determined by the dimensions of the molds employed.

The invention is further illustrated by the following examples.

## EXAMPLE 1

### Inoculum Preparation

A typical medium used to grow the various stages of inoculum is prepared according to the following formula:

| | |
|---|---|
| Dextrose | 1.0% |
| Dextrin | 2.0% |
| Yeast extract | 0.5% |
| NZ amine | 0.5% |
| Calcium carbonate | 0.1% |
| Water qs | to 100% |

This medium is sterilized. A 100 ml portion of this sterile medium, in a flask, is inoculated with mycelial scrapings from an agar slant of Streptomyces cyaneogriseus noncyanogenus NRRL 15773. The medium is then agitated vigorously on a rotary shaker for 48-72 hours at 28 °C providing primary inoculum. This primary inoculum is then used to inoculate one liter of the above sterile medium, which is then grown aerobically at 28 °C for 48 hours providing secondary inoculum.

EXAMPLE 2

Fermentation

A fermentation medium of the following formulation is prepared.

| | |
|---|---|
| Dextrin | 1.0% |
| Soya peptone | 1.0% |
| Molasses | 2.0% |
| Calcium carbonate | 0.1% |
| Water qs | to 100% |

This medium is sterilized and then a 30 liter portion is inoculated with one liter of secondary inoculum prepared as described in Example 1. The fermentation is conducted at 30 °C, with a sterile air flow of 30 liters per minute, backpressure of 0.562 kg/$cm^2$ (8 psig) and agitation by an impeller operated at 500 rpm for 91 hours at which time the mash was harvested.

**EXAMPLE 3**

**Isolation of LL-F28249α**

A total of 26 liters of whole harvest mash, prepared as described in Example 2 is mixed with 1500 g of diatomaceous earth and filtered. The mycelial cake is washed with 5 liters of water and the filtrate and wash discarded. The mycelial cake is mixed with 10 liters of methanol for one hour, then filtered and washed with 5 liters of methanol. The methanol extract and methanol wash are combined and evaporated to an aqueous residue of about 1-2 liters. This aqueous residue is mixed with twice its volume of methylene chloride and mixed for 1/2 hour. The methylene chloride phase is separated and then concentrated to a syrup giving 27 g of crude material.

This 27 g of crude material is dissolved in a mixture of methylene chloride and methanol, filtered through cotton and anhydrous sodium sulfate and then evaporated, giving 7.0 g of an oil.

A 170 g portion of silica gel is slurried in 12.5% ethyl acetate in methylene chloride and poured to form a column 2.5x58 cm. The oil is dissolved in 12.5% ethyl acetate in methylene chloride and applied to the column. The column is developed with the same solvent mixture. The mobile phase is run at 1.3 ml/minute initially and 15 minute fractions are collected. The flow rate slowed to about 0.5 ml/minute after 10 fractions, so fractions 1-10 are 20 ml decreasing to about 10 ml uniformly and fractions 11-98 were about 7 ml. At fraction 99 the flow rate is increased to give 25 ml fractions in 10 minutes. A total of 105 fractions are collected. These fractions were tested by thin layer chromatography in ethyl acetate:methylene chloride (1:1).

Fractions 55-62 are combined and evaporated giving 150 mg of solid containing LL-F28249α and β.

The 150 mg of solid containing LL-F28249α and β are chromatographed by preparative HPLC using a reverse-phase column (Whatman C8, 2.2x50 cm) developed with 80% (v/v) methanol in water. The flow rate is about 10 ml/minute and 2 minute fractions are collected.

Fractions 58-69 are combined, the methanol is evaporated, t-butanol is added and the mixture is lyophilized, giving 60 mg of pure LL-F28249α.

This compound may be illustrated as follows:

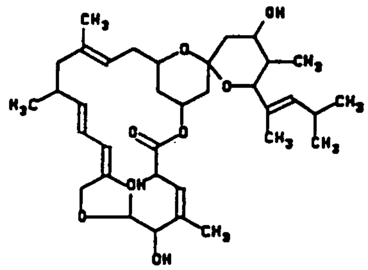

**EXAMPLE 4**

**5-O-t-Butyldimethylsilyl-LL-F28249α**

In 500 Ml of $CH_2Cl_2$, 70 g of LL-F28249α is stirred with 82.04 g of imidazole at 20°C under $N_2$ atmosphere. Then, 43 g of t-butyldimethylsilyl chloride in 400 mL of $CH_2Cl_2$ is added over 5 minutes. After an hour, the reaction is assayed for completion by high performance liquid chromatography (HPLC), using 50% $CH_3CN$/50% $H_2O$ in a curved gradient mode over 10 minutes on a Whatman Partisil CCS/$C_8$ rapid analysis column at 1 mL/min flowrate. Another 3 g of t-butyldimethylsilyl chloride is added, and after 3 hours the composition is 92.3% product, 0.3% LL-F28249α and 1.16% disilylated material. The mixture is diluted with $CH_2Cl_2$ and poured into 2 L of $H_2O$. The $CH_2Cl_2$ layer is separated. The aqueous portion is extracted with 2 L of $CH_2Cl_2$, and the combined organic layers are dried ($Na_2SO_4$). The $CH_2Cl_2$ is evaporated in vacuo to afford 166 g of the title compound that is identified by mass spectrometry and nuclear magnetic resonance (NMR) spectrometry.

**EXAMPLE 5**

**5-O-t-Butyldimethylsilyl-23-oxo-LL-F28249α**

In 5 L of dry $CH_2Cl_2$, 116 g of 5-O-t-butyldimethylsilyl LL-F28249α is stirred under $N_2$, and 540 g of NaOAc is added at 22°C, followed by addition of 172.5 g of pyridinium chlorochromate (PCC). After 1 hour, an additional 15 g of PCC is added since the reaction is incomplete by HPLC analysis. After 2 hours, another 10 g of PCC is added, and the reaction is stirred for a total of 5 hours. The mixture is poured into 6 L of ice-water mixture, and the $CH_2Cl_2$ is separated. The aqueous layer is extracted with $CH_2Cl_2$, and the combined $CH_2Cl_2$ layers are washed with water and dried ($Na_2SO_4$). The $CH_2Cl_2$ is evaporated in vacuo to afford 197.8 g of crude product, which is dissolved in 2 L of $Et_2O$ and filtered. The $Et_2O$ solution is washed with water (2 x 1000 mL), dried ($Na_2SO_4$) and evaporated to dryness to give 60 g of the title compound which is identified by mass spectrometry and NMR spectroscopy.

The pyridinium chlorochromate substituted with pyridinium dichromate in the above procedure also affords the title compound.

**EXAMPLE 6**

**23-Oxo-LL-F28249α**

In 1.5 L of MeOH, 60 g of 5-O-t-butyldimethylsilyl-23-oxo-LL-F28249α is dissolved by warming, and at 0°C, 30 g of p-toluenesulfonic acid in 300 mL of MeOH is added. The mixture is stirred for 3 hours and poured into 6 L of saturated $NaHCO_3$ solution in 6 L of $H_2O$. After stirring, the mixture is extracted with 4 L of EtOAc, and the layers are separate. The aqueous layer is saturated with NaCl and extracted with 2 x 6 L of EtOAc. The first EtOAc layer is washed with saturated NaCl solution, combined with the other EtOAc extracts and dried ($Na_2SO_4$). The EtOAc is evaporated in vacuo to afford 148.1 g of dark residue. The crude material is then chromatographed by HPLC on 1200 g of $SiO_2$ using 1% isopropanol in $CH_2Cl_2$ to elute and

monitored by an ultraviolet detector/254 nM filter. Fractions 39-42 are combined and evaporated to dryness to afford 12.65 g of the title compound which analyzes as follows:

| | | |
|---|---|---|
| Anal. Calcd for $C_{36}H_{50}O_8$: | C, 70.79; | H, 8.25 |
| Found: | C, 70.33; | H, 8.31 |

The title compound is further identified by mass spectrometry and NMR spectroscopy.

## EXAMPLE 7

### 23-O-Methyloxime-LL-F28249α

In 930 mL of dry dioxane at room temperature, 70 g of 23-oxo-LL-F28249α, 11.8 g of NaOAc, 11.8 g of $CH_3ONH_2 \cdot HCl$ and 2.1 mL of HOAc are added. The mixture is stirred under $N_2$ for 3 days, and after no starting material is detected by HPLC, 650 mL of dioxane is evaporated in vacuo. The residue is poured into 5 L of $H_2O$, and the product is extracted with $CH_2Cl_2$ (4 x 2 L). The combined extracts are washed with $H_2O$, dried ($Na_2SO_4$ and evaporated to dryness. The residue is dissolved in 1500 mL of $Et_2O$, and the solution is washed with $H_2O$, dried ($Na_2SO_4$) and evaporated to dryness. This gives 11.84 g of the title compound, which is identified by mass spectrometry and NMR spectroscopy. It also analyzes as follows:

| | | | |
|---|---|---|---|
| Anal. Calcd for $C_{37}H_{53}O_8N \cdot 1.5\ H_2O$: | C, 66.64; | H, 8.46; | N, 2.10 |
| Found: | C, 66.82; | H, 8.13; | N, 2.32 |

This compound may be illustrated as follows:

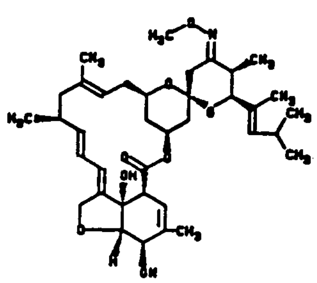

## EXAMPLE 8

### Preparation of Sustained Release Bolus

Boluses are made according to the following formulation:

| Ingredient | % |
|---|---|
| 23-(O-methyloxime)LL-F28249α (89% real) | 0.34 |
| Glycerol Monostearate | 15.95 |
| Carnauba Wax | 3.96 |
| Barite | 79.75 |

The monostearate and carnauba wax are blended, melted and heated to 105 ± 5 deg. C. To this melt is added the active ingredient and blended to a uniform suspension. To this suspension is added the barite with continual mixing. Blending continues until a uniform creamy mixture is obtained. The resulting material is poured into a mold, allowed to cool to room temperature and demolded. The resulting boluses weigh between 51 and 53 grams. Each bolus is domed at both ends and 19.05 mm (3/4 in.) thick by 22.22mm (7/8 in.) wide by 76.2 mm (3.0 in.) long.

Specifications for glyceral monostearate indicate that this material should be a white powder or beads essentially free of foreign matter. The particle size should not exceed 5% on a U. S. Standard Sieve No. 20 and have an acid value of 2-3.0 and a moisture content of not more than 1.5%. Total monoglycerides should exceed 40%, but free acid should not exceed 1.5%.

Carnauba wax should be a yellow powder essentially free of foreign matter. 100% of the wax should pass through a U. S. Standard Sieve No. 80. The acid number of the wax should be 2.0 - 6.0 and acetone soluble resinous matter at 15 °C should not exceed 5.0%.

The barite should be a gray powder essentially free of foreign matter and having a bulk density of about 2.40 g/cm$^3$ (150 pounds per cubic foot). The particle size should be such that not more than 4% is retained on a No. 200 U. S. Standard Sieve and not less than 80% pass through a No. 325 U. S. Standard Sieve. The chemical analysis for the acceptable product is $BaS0_4$ 90 - 94%; $Si0_2$ 7 - 8% and $Fe_20_3$ 0.1 - 0.2%.The density of the boluses of this invention should be between about 2.35 and 2.6 ± 0.6.

Other bolus compositions are prepared in the same manner and are reported in Table I below.

**Table I**

**Slow Release Bolus Compositions**

| **Ingredient** | **A** | **B** | **C** | **D** | **E** | **F** | **G** | **H** |
|---|---|---|---|---|---|---|---|---|
| 23-(O-methyloxime) LL-F28249 (89% real) | 0.34 | 1.69 | 8.44 | 1.70 | | | | |
| 23-(O-methyloxime) LL-F28249 (90% real) | | | | | 1.63 | 3.26 | 4.90 | 6.50 |
| Glycerol monostearate | 15.95 | 15.73 | 14.65 | 15.70 | 14.40 | 14.40 | 14.40 | 14.40 |
| Carnauba wax | 3.96 | 3.93 | 3.66 | 3.90 | 3.60 | 3.60 | 3.60 | 3.60 |
| Barite | 79.75 | 78.65 | 73.25 | 78.70 | 80.37 | 78.74 | 77.10 | 75.50 |
| | 100.0 | 100.0 | 100.0 | 100.0 | 100.0 | 100.0 | 100.0 | 100.0 |

**EXAMPLE 9**

**Determination of Erosion rates of Boluses of the present invention**

The drug release rates for boluses designated A, B and C in Table I are determined by initially assaying said boluses, for the compound 23-(O-Methyloxime)LL-28249α, which are to be administered to the calves selected for the test. These assays show that boluses which are to be evaluated contain 0, 713, 1035 or 1260 mg of 23-(O-Methyloxime)LL-28249α. The 50-53 gram boluses are broken in half, weighed and orally administered, one 1/2 bolus to each calf. The treated calves are then penned and fed and watered according to conventional or standard cattle raising procedures. The diet for all animals is a standard cattle ration.

At intervals of 70, 119 and 167 days after administration, four animals from each treatment group are necroposied and the boluses retreived and examined to determine whether the surface is rough or smooth and then weighed. The results are thereafter recorded and evaluated. This procedure is repeated at 119 days after treatment for a second group of animals from each treatment and then repeated again at 167 days with a third group of animals from each treatment group.

For convenience, the results obtained from each treatment group, four calves per group, are averaged and reported in Table II.

Table II

**Erosion Rate of Boluses A, B and C from Table I**

| Average Bolus Assay 23-(O-methyloxime) LL-F28249alpha (mg) | Average Initial Bolus weight (mg) | Day Bolus Recovered | Average mg/hd/day delivery of 23-(O-methyloxime) LL-F28249alpha (mg) | Average animal weight for the period (kg) | Release rate mg/kg/day of 23-(O-methyloxime) LL-F28249alpha delivered from day 1 until recovery of Bolus | Average release rate mg/kg/day of 23-(O-methyloxime) LL-F28249alpha delivered between days 70 to 119 and 119 to 167 |
|---|---|---|---|---|---|---|
| 713 | 26.7 | 70 | 6.07 | 113.4 | 0.054 | - |
| 713 | 26.7 | 119 | 4.95 | 119.6 | 0.041 | 0.028 |
| 713 | 27.1 | 167 | 3.45 | 122.4 | 0.028 | 0.0014 |
| 1035 | 26.2 | 70 | 8.59 | 121.8 | 0.071 | - |
| 1035 | 26.7 | 119 | 6.19 | 111.0 | 0.056 | 0.025 |
| 1035 | 26.1 | 167 | 5.31 | 129.7 | 0.041 | 0.0 |
| 1260 | 25.8 | 70 | 10.41 | 129.0 | 0.081 | - |
| 1260 | 25.6 | 119 | 6.67 | 113.3 | 0.059 | 0.012 |
| 1260 | 25.8 | 167 | 7.09 | 129.2 | 0.055 | 0.06 |

EXAMPLE 10

Preparation of a sustained release Bolus formulation and evaluation thereof for control of Gastrointestinal Helminths and Boophilus microplus ticks on cattle

Sustained release bolus formulations consisting essentially of 23-(O-methyloxime)LL-F28249α, glycerol monostearate, carnauba wax and barite are prepared by blending the appropriate amounts of each ingredient in a blender maintained at about 80°C for 10 minutes.

After blending, the mixture is discharged and transferred to a pelletizing extruder and/or directly charged to the injection molder.

If pelletizing is carried out, this mixture is then pelletized using a bell shaped temperature profile that has a cooled feed section (50-60°C), hot transition section (100-110°C) and a cooled metering section (5-60°C) with a straight-through 3.175 mm (1/8 inch) die equipped with a (20 mesh) screen with 0.84 mm openings breaker plate. The die temperature is maintained at 55-60°C. Resulting extruded rope is air cooled (air stream blowing at the die opening) and cut using a hot face cutter to 6.35 mm (1/4" inch) long pellets.

In the case of direct feeding of the dry blend, a feeding system such as a Vibra-Screen may be used, to transfer the material to an injection molding system utilizing a hot runner system and a shut-off nozzle. Operating conditions for the injection molder should be the bell shaped temperature profile described for the pelleting operation above, e.g. fill time (10-20 sec.), injection time (10-20 sec.), injection pressure 70.3 kg/cm$^2$ (0.5 tons psig) and hold time (5-10 sec.) yielding boluses weighing about 51 grams each and having a density of about 2.5 g/cc and a hardness as measured on a Delamar Press of about 77 to 80 kg.

Boluses prepared by this method are shown below.

**Bolus Compositions Prepared By Injection Molding**

| | % by weight | | | |
|---|---|---|---|---|
| **Ingredient** | **Bolus I** | **J** | **K** | **L** |
| 23-(O-methyloxime) LL-F28249α | 0.0 | 3.24 | 4.84 | 6.46 |
| Glycerol monostearate | 14.40 | 14.40 | 14.40 | 14.40 |
| Carnauba wax | 3.60 | 3.60 | 3.60 | 3.60 |
| Barite | 82.00 | 78.76 | 77.16 | 75.54 |
| | (weight in grams) | | | |
| 23-(O-methyloxime) LL-F28249α | 0 | 28.49 | 42.15 | 26.49 |
| Glycerol monostearate | 125.28 | 125.28 | 125.28 | 59.04 |
| Carnauba wax | 31.32 | 31.32 | 31.32 | 14.76 |
| Barite | 713.60 | 685.21 | 671.29 | 309.71 |

In order to evaluate the above-identified compositions for the control of gastrointestinal helminths and cattle tick infestations in or on cattle, the following tests are conducted.

Fifty-two abedeen angus calves, (less than one year of age) and on pasture, are selected for evaluation of the boluses described above and designated I, J, K and L.

The boluses contain 0, 713, 1035 or 1260 mg of 23-(O-methyloxime)LL-F28249α, respectively, per 1/2 bolus each of which weighs about 25 grams.

During the week before treatment two separate differential nematode egg counts are done on the calves and the severity of tick infestations assessed. The animals are ear tagged, weighed and assigned to one of four treatment groups, 12 calves per group. A fifth group of 4 animals is also ear tagged and weighed. This group is used to study the feasability of reintroducing boluses recovered after necropsy into other animals.

Differential nematode egg counts are done one week, two weeks, 1 month and monthly for 5 months or until there is no longer adequate control.

Data obtained are reported in Tables III and IV below where it can be seen that control animals, untreated for the first 67 days of the trial, remain severely infested throughout this portion of trial period. These animals are then treated with levamisole and the nematode infestations are very markedly reduced. Animals receiving the boluses of this invention containing from 713 mg to 1260 mg of 23-(O-methyloxime)-LL-F28249α are essentially free of nematode infestation from 28 days post treatment through 167 days post treatment.

Ticks are eliminated within one week of treatment with the boluses containing 1035 mg of 1260 mg of 23-(O-methyloxime)LL-F28249α. Animals receiving boluses containing 713 mg of the active ingredient take two weeks to be cleared of ticks. The control animals have to be dipped in a tickicide solution to prevent mortality. The treated animals remain free of ticks for 97 days and until the cold weather sets in which eliminated tick infestations on control animals.

Table III

Evaluation of Boluses containing 23-(o-methyloxime)LL-F28249alpha for prolonged control of nematode infestations of cattle

| Days Post-Treatment | 0 mg Bolus Animals WB/Tl | 0 mg Bolus Ave. EPG | 713mg Bolus Animals WB/Tl | 713mg Bolus Ave. EPG | 1035mg Bolus Animals WB/Tl | 1035mg Bolus Ave. EPG | 1260mg Bolus Animals WB/Tl | 1260mg Bolus Ave. EPG |
|---|---|---|---|---|---|---|---|---|
| 0 | 12/12 | 519 | 12/12 | 873 | 12/12 | 552 | 12/12 | 581 |
| 28 | 11/11 | 1041 | 12/12 | 0 | 12/12 | 0 | 12/12 | 0 |
| 44 | 11/11 | 1465 | 12/12 | 0 | 12/12 | 0 | 10/10 | 0 |
| 65 | 10/10# | 2043 | 12/12 | 0 | 12/12 | 0 | 10/10 | 0 |
| 97 | 7/7 | 108 | 8/8 | 0 | 8/8 | 0 | 6/6 | 0 |
| 116 | 7/7 | 308 | 7/8 | 25 | 8/8 | 0 | 6/6 | 0 |
| 140 | 3/3 | 317 | 4/4 | 25 | 5/5 | 0 | 3/3 | 0 |
| 167 | 2/3 | 717 | 4/4 | 0 | 3/5 | 0 | 1/3 | 100 |

WB/Tl = with boluses/total animals
# Control animals treated with levamisole (3.75mg/kg) on day 67 of the trial
EPG = Nematode eggs per gram of feces

Table IV

**Evaluation of Boluses containing 23-(O-methyloxime)LL-F28249alpha for prolonged control of nematode populations in cattle**

| NECROPSY DAY POST TREATMENT | BOLUS CONC. MG* | NO. ANIMALS W BOLUS/TOTAL | PER CENT REDUCTION OF HAEMONCHUS ADULT | PLACEI LARVAE | OSTERTAGIA ADULT | OSTERTAGI LARVAE | COOPERIA ADULT | PUNCTATA LARVAE | OESOPHAGOSTOMUM ADULT | DICTYOCAULUS ADULT |
|---|---|---|---|---|---|---|---|---|---|---|
| 70 | 1260 | 4/4 | 100 | 100 | 100 | | 100 | - | 100 | 100 |
| | 1035 | 4/4 | 100 | 100 | 100 | | 99.9 | - | 100 | 100 |
| | 713 | 4/4 | 100 | 100 | 100 | | 100 | - | 100 | 100 |
| | 0** | 4/4 | 4617 | 542 | 200 | | 35783 | 0 | 665 | 67 |
| | | | (4000-6167) | (333-667) | (66-533) | | (19600-51033) | | (310-1072) | (5-118) |
| 119 | 1260 | 3/3 | 100 | 100 | 100 | 100 | 100 | 100 | 100 | 100 |
| | 1035 | 3/3 | 100 | 100 | 100 | 100 | 100 | 100 | 100 | 100 |
| | 713 | 3/4 | 100 | 100 | 100 | 100 | 98.8 | 97.2 | 100 | 100 |
| | 0** | 3/3 | 1752 | 674 | 441 | 74 | 23959 | 1322 | 228 | 120 |
| | | | (500-2400) | (67-1155) | (67-756) | (0-133) | (18778-32500) | (433-2533) | (60-400) | (0-319) |
| 167 | 1260 | 1/3+ | 98.5 | 97.3 | 92.8 | | 73.4 | 100 | 100 | 100 |
| | 1035 | 3/5 | 99.3 | 100 | 100 | | 99.4 | 100 | 100 | 100 |
| | 713 | 4/4 | 100 | 100 | 100 | | 99.5 | 100 | 100 | 100 |
| | 0** | 2/3 | 2067 | 617 | 10130 | | 29567 | 400 | 413 | 2 |
| | | | (1467-2533) | (533-1881) | (3200-22898) | | (14033-59667) | (33-933) | (88-880) | (0-5) |

* Per head (In a half bolus [25+ grams])
** Number of nematodes: Numbers in ()'S give the range
\+ Animal with bolus had no worms

## EXAMPLE 11

### Evaluation of Slow Release Boluses for Prophylactic control of psoroptic mange on cattle

Boluses containing 375 mg of 23-(O-methyloxime)LL-F28249α per 1/2 bolus, are prepared by the injection molding technique described in Example 10. The boluses prepared have the following composition.

| Ingredient | % by weight | Grams |
|---|---|---|
| 23-(O-methyloxime)LL-F28249α | 1.53 | 1.61 |
| Glycerol monostearate | 15.73 | 16.52 |
| Carnauba wax | 3.93 | 4.12 |
| Barite | 78.81 | 82.75 |
| | 100.00 | 105.00 |

Cattle receiving the boluses are given one half of a 50 gram bolus containing 750 mg of 23-(O-methyloxime)LL-F28249α.

Mixed heifers are selected for the trial. The animals are placed in a dry lot and started on a health program to protect against common disease problems. They are ear tagged and placed in stanchions to prevent licking the mange infestation.

Live mites are then transferred to each animal. The transfers are made by taking scrapings from heavily infested donor animals and transferring them to candidate animals.

Four uninfested animals are randomly selected from animals which have not been placed in the barn. These four animals later receive the bolus treatment and are infested one week later. Mites are placed on each of these animals, six times during the next two weeks.

Care is taken to prevent cross contamination between animals. Scraping are made at weekly intervals and evaluated the same day.

The mites are counted by stage, using a 20-30x dissecting microscope. The 375 mg bolus prevented the Psoroptic mange infestation from establishing on the cattle receiving six heavy challenges of mites.

Data obtained are reported in Table V below.

**Table V**

**Average number of live mites (Psoroptes ovis) per animal**

| Treatment | 1 day prior to Treatment | Days Post-Treatment 8 | 16 | 22 | 29 | 34 | 43 | 47 | 56 |
|---|---|---|---|---|---|---|---|---|---|
| Bolus containing | XX | - | - | 0 | 0 | 0 | 0 | 0 | 0 |
| 375 mg 23-(O-methyloxime) | XX | - | - | 0 | 0 | 0 | 0 | 0 | 0 |
| LL-F28249alpha | XX | - | - | 0 | 0 | 0 | 0 | 0 | 0 |
| | XX | - | - | 0 | 0 | 0 | 0 | 0 | 0 |
| Untreated Control | 274 | 130 | 421 | 757 | 285 | 464 | deceased | | |
| | 123 | 149 | 50 | 403 | 218 | deceased | | | |
| | 69 | 66 | 39 | 20 | 6 | 27 | 14 | 16 | 14 |
| | 1 | 4 | 3 | 9 | 1 | 5 | 9 | 16 | 16 |

XX = Bolus group was not infested until 1 week after test initiated; and then it was found that mite colonies could not be established on bolus treated animals. Counts based on 2 scrapings/animal/sample period

— = mite colony could not be established

**EXAMPLE 12**

**Evaluation of Boluses containing 23-(O-methyloxime) LL-F28249α for the sucking lice and face flies on cattle**

The Boluses used in these evaluations are prepared by an injection molding technique as described in Example 10. The prepared boluses have the following compositions.

| Bolus | I | | II | | III | |
|---|---|---|---|---|---|---|
| **Ingredient** | **% by wt.** | **grams** | **% by wt.** | **grams** | **% by wt.** | **grams** |
| 23-(O-methyloxime LL-F28249α | 1.62 | 6.64 | 3.24 | 28.49 | 4.84 | 42.15 |
| Glycerol Monostearate | 14.40 | 59.04 | 14.40 | 125.28 | 14.40 | 125.28 |
| Carnauba wax | 3.60 | 14.76 | 3.60 | 31.32 | 3.60 | 31.32 |
| Barite | 80.38 | 329.56 | 78.76 | 685.21 | 77.16 | 671.29 |

For evaluation the boluses are broken in half.
Bolus I Contains 375 mg AI/half bolus
Bolus II Contains 750 mg AI/half bolus
Bolus III Contains 1125 mg AI/half bolus
AI = active ingredient

Prior to treatment all animals are weighed, ear tagged, and inspected for lice. The number of lice are estimated from visual inspection of 2-3 cm hairparts from each of the following locations: ear, eyes, muzzle, jaw, brisket, dewlap, shoulder, backline, tailhead, and hips. Animals are randomly assigned to groups including 3 treatments and 1 control of 5 animals each. Groups I, II, and III are given boluses containing the experimental compound while group 1 is untreated. All animals are confined to 2 acre lots and given free access to alfalfa hay and mineral supplement. Lice on the animals are assessed at days 7, 14, 28, and bimonthly throughout the test.

Lice estimates are made while the animals are restrained in a head gate under artificial lighting.

Fecal samples (app. 10 g) are taken for nematode analysis on days 7, 28, and monthly thereafter. Samples are stored at -10°C.

All bolused treatment groups (375 mg, 750 mg and 1125 mg) of animals have significantly reduced levels of the blue cattle louse, Solenoptes capillatus at 1 week post-treatment.

Complete control of sucking lice (Solinoptes capillatus) on all bolus treated cattle is achieved for 100 days.

**EXAMPLE 13**

**Evaluation of a Slow Release Bolus for controlling Hypoderma spp. in cattle for a prolonged period**

Boluses used in this evaluation are prepared by injection molding as described in Example 10 above. The boluses evaluated have the following composition:

| **Ingredient** | **by wt.** | **grams** |
|---|---|---|
| 23-(O-methyloxime)LL-F28249α | 1.6 | 3.2 |
| Glycerol monostearate | 14.4 | 28.8 |
| Carnauba wax | 3.6 | 7.2 |
| Barite | 80.4 | 160.8 |
| | 100.0 | 200.0 |

In these tests mixed breed cattle naturally infested with first instar Hypoderma spp. are randomly divided in treatment groups of 4 calves each. One group serves as untreated controls another group receives one half of a 50-54 gram bolus containing 375 mg of 23-(O-methyloxime)LL-F28249α. The half boluses are administered to the calves with a balling gun.

After administration efficacy is determined through visual examination and palpation of the backs of the calves, on a weekly basis, for presence of warbles. Treatment with the 23-(O-methyloxime)LL-F28249α boluses are complete effective against Hypoderma spp. No grubs appear in the backs of cattle receiving the 375mg boluses containing the above-said compound over the 72 day trial; however, untreated control animals average 12 (range 4-19) grubs/head at the peak count at 58 days and 8.8 at 72 days.

## EXAMPLE 14

### Evaluation of Boluses containing from 75 to 1875 mg of LL-F28249α for prolonged control of nematodes and ticks on cattle

The boluses for evaluation are prepared as follows:

The monostearate and carnauba wax are blended, melted and heated to 105 ± 5 °C. To this melt is added the active ingredient and blended to a uniform suspension. To this suspension is added the barite with continual blending. Blending continues until a uniform creamy mixture is obtained. The resulting material is poured into a mold, allowed to cool to room temperature and demolded. The resulting boluses weigh between 51 and 53 grams. Each bolus is domed at both ends and 19.05 mm (3/4 in.) thick by 22.22 mm (7/8 in.) wide by 76.2 mm (3.0 in.) long.

One half boluses (weight 25.5 to 27.0 g) and are administered to cattle on pasture. Efficacy is monitored over 120 days by determining the number of internal and external parasites.

| Ingredient | % by wt | gms | % by wt | gms | % by wt | gms |
|---|---|---|---|---|---|---|
| LL-F28249α | 0.34 | 5.1 | 1.69 | 25.35 | 8.44 | 126.6 |
| Glycerol monostearate | 15.95 | 239.25 | 15.73 | 235.95 | 14.65 | 219.75 |
| Carnauba wax | 3.96 | 59.40 | 3.93 | 58.95 | 3.66 | 54.90 |
| Barite | 79.75 | 1196.25 | 78.65 | 1178.75 | 73.25 | 1098.75 |

Bolus I contains 75 mg LL-F28249α per half bolus, bolus II contains 375 mg LL-F28249α per half bolus and bolus III contains 1875 mg LL-F28249α per half bolus.

In this test pastured animals are randomly divided into groups of 5 animals per group and ear-tagged. The feces of all animals is examined prior to the tests in order to determine the average number of nematode eggs per gram of feces of the test animals. All calves appear to have between 2700 and 3000 eggs per gram of feces. The test is begun with all animals receiving one half of a bolus weighing about 25 to 27 grams. Control animals receive 3.75 mg/kg of body weight of levamisole at the start of the test and on day 62; other treatment groups receive half boluses I, II or III as described above containing 75, 375 or 1875 mg of LL-F28249α, respectively. The animals are then placed in individual paddocks and examined at intervals during the holding period up to 120 days. On day 7, 16, 32, 62, 91 and 106 following treatment, feces are collected from each animal and the average number of nematode eggs per gram of feces is determined. Data obtained show that the 75 mg bolus reduced the nematode egg counts and nematode burdens to levels below the two treatments with levamisole. The 375 mg and 1875 mg bolus gave 99.9% reduction of nematode eggs within one week and gave 99.9% control of nematodes. The superior weight performance of animals receiving the 375 and 1875 mg boluses were highly significant statistically. The weight of animals receiving the 75 mg bolus was equivalent to the levamisole control.

**Table VI**

**Evaluation of boluses containing 75, 375 and 1875 mg of LL-F28249alpha for control of nematodes in cattle**

| | | Average number of nematode eggs per gram of feces | | | |
|---|---|---|---|---|---|
| **Treatment** | **# Calves** | **Days 0** | **7** | **62** | **119** |
| (Recommended dose) Levamisole day 0 and day 62 | 10 | (2850) | 5 (99.9%) | (Levamisole) 755 | 146 |
| Bolus I (75 mg) | 10 | (2850) | 940 (67%) | 205 | 5 |
| Bolus II (375 mg) | 10 | (2850) | 0 (100%) | 0 | 5* |
| Bolus III (1875 mg) | 10 | (2850) | 0 (100%) | 0 | 5** |

* 1 Animal had 25 EPG

** 6 Animals with bolus had 0 EPG's; 4 animals without bolus had positive EPG'S

**TABLE VII**

**EFFICACY OF 11-F28259 BOLUS AGAINST NEMATODES IN CATTLE**

**% CONTROL OF NEMATODES**

| Treatment | # Cattle | Haemonchus | Ostertagia | Cooperia | Oesophlogostonum |
|---|---|---|---|---|---|
| 75 mg bolus | 10 | 92 | 100 | 0 | 25 |
| 375 mg bolus | 10 | 100 | 100 | 97 | 99+ |
| 1875 mg bolus | 10 | 100 | 100 | 99+ | 100 |
| Levamisole Control Avg. number worms (dewormed 57 days earlier) | 10 | 2325 | 729 | 5937 | 117 |

Table VIII

Weight gains of cattle receiving levamisole 3.75 mg/kg on day 0 and day 62 and from boluses containing LL-F28249alpha and delivering 0.37 mg/hd/day, 1.96 mg/hd/day or 3.76 mg/hd/day of LL-F28249alpha

| Treatment | Average weight gains (Kg) from Day 0 | | |
|---|---|---|---|
| | Days 7 | 62 | 110 |
| Levamisole (3.75 mg/kg on day 0 and day 62) | 7 | 0 | 21 |
| Bolus I 75 mg (0.0031 mg/kg/day) | 5 | 7 | 24 |
| Bolus II 375 mg (0.016 mg/kg/day) | 6 | 11 | 37 |
| Bolus III 1875 mg (0.031 mg/kg/day) | 8 | 15 | 40 |

## Claims

1. A sustained release bolus for oral administration to ruminant animals, effective for protecting said ruminant animals for a prolonged period of time against adult and larval infestation by helminths,

endoparasitic insects, ectoparasitic insects and acarids, comprising 0.3% to 10% by weight of LL-F28249α, 23-(O-methyloxime)LL-F28249α or derivative thereof; 10% to 20% by weight of glycerol monostearate; 3.0% to 10.0% by weight of carnauba wax and 70.0% to 85% by weight of barium sulfate or barite fines.

2. The sustained release bolus according to Claim 1 comprising 1.0% to 8.5% by weight of said 23-(O-methyloxime) LL-F28249α or LL-F28249α, 14% to 16% by weight of glycerol monostearate, 3.0% to 5.0% by weight of carnauba wax and 70% to 80,5% by weight of barite.

3. The sustained release bolus according to Claim 1 effective for the control or prevention of helminth infestations in ruminant animals comprising 0.3% to 1.0% by weight of LL-F28249α or 23-(O-methyloxime)LL-F28249α, 14% to 16% by weight of glycerol monostearate, 3.0% to 5.0% by weight of carnauba wax and 78.01% to 82.7% by weight of barium sulfate or barite fines.

4. A bolus according to Claim 1 effective for treatment of ruminant animals to protect them against infestation by helminths, endoparasitic insects, ectoparasitic insects and acarids, for from 120 to 180 days, wherein said bolus continously releases into the digestive track of said ruminant animals 0.04 mg/head/day to 15.63 mg/head/day of LL-F28249α or 23-(O-methyloxime)LL-F28249α.

5. A bolus according to Claim 4, wherein said bolus releases into the digestive track of said ruminant animals 0.2 mg/head/day to 10.4 mg/head/day of LL-F28249α or 23-(O-methyloxime)LL-F28249α.

6. The bolus according to Claim 1 for preventing or inhibiting nematode and tick infestation of cattle for up to 167 days comprising from 75 mg to 1875 mg of LL-F28249α or 23-(O-methyloxime)LL-F28249α dispersed in a mixture comprising 14.00% to 16% by weight glycerol monostearate, 3.00% to 5.0% by weight of carnauba wax and 70.00% to 80.5% by weight of barium sulfate or barite fines.

7. A bolus according to Claim 6, wherein said bolus releases into the digestive track of said ruminant animals from 0.001 mg/kg of animal body weight per day to 0.075 mg/kg of animal body weight per day of LL-F28249α or 23-(O-methyloxime) LL-F28249α.

8. A bolus according to Claim 1 for the systemic control of ectoparasitic insect infestations of ruminant animals, wherein said bolus releases into the rumen of the treated animal a systemically effective amount of LL-F28249α, 23-(O-methyloxime)LL-F28249α or derivative thereof.

9. A bolus according to Claim 8 comprising about 375 mg to 1875 mg of LL-F28249α or 23-(O-methyloxime) LL-F28249α, 14% to 16% by weight of glycerol monostearate, 3% to 5% by weight of carnauba wax and 70% to 80% by weight of barite.

10. A bolus for reducing or preventing contamination of pastures of ruminant animals by the infective stages of nematodes, endoparasitic insects, ectoparasitic insects and acarids that infest said animals, comprising 0.3% to 10% by weight of LL-F28249α or 23-(O-methyloxime)LL-F28249α; 10% to 20% by weight of glycerol monostearate; 3.0% to 10% by weight of carnauba wax and 70.0% to 85% weight of barium sulfate or barite fines, whereby said bolus kills said endo- and ectoparasites and/or inhibits their reproductive cycle, thus eliminating the eggs of said parasites from the feces of the treated animals.

**Patentansprüche**

1. Bolus mit lang andauernder Abgabe zur oralen Verabreichung an Wiederkäuer, wirksam zum Schutz der Wiederkäuer für eine verlängerte Zeitdauer gegen Befall durch ausgewachsene Helminthen, endoparasitäre Insekten, ektoparasitäre Insekten und Acarida sowie deren Larven, umfassend 0,3 Gew.-% bis 10 Gew.-% von LL-F28249α, 23-(O-methyloxim)LL-F28249α oder ein Derivat davon, 10 Gew.-% bis 20 Gew.-% Glycerinmonostearat, 3,0 Gew.-% bis 10,0 Gew.-% Carnaubawachs und 70 Gew.-% bis 85 Gew.-% feinteiliges Bariumsulfat oder Baryt.

2. Bolus mit lang andauernder Abgabe nach Anspruch 1, umfassend 1,0 Gew.-% bis 8,5 Gew.-% des 23-(O-methyloxim)LL-F28249α oder LL-F28249α, 14 Gew.-% bis 16 Gew.-% Glycerinmonostearat, 3,0 Gew.-% bis 5,0 Gew.-% Carnaubawachs und 70 Gew.-% bis 80,5 Gew.-% Baryt.

3. Bolus mit lang andauernder Abgabe gemäß Anspruch 1, wirksam zur Bekämpfung oder Vorbeugung von Helminthenbefall in Wiederkäuern, umfassend 0,3 Gew.-% bis 1,0 Gew.-% von LL-F28249α oder 23-(O-methyloxim)LL-F28249α, 14 Gew.-% bis 16 Gew.-% Glycerinmonostearat, 3,0 Gew.-% bis 5,0 Gew.-% Carnaubawachs und 78,01 Gew.-% bis 82,7 Gew.-% feinteiliges Bariumsulfat oder Baryt.

4. Bolus nach Anspruch 1, wirksam zur Behandlung von Wiederkäuern, um sie gegen Befall durch Helminthen, endoparasitäre Insekten, ektoparasitäre Insekten und Acarida für eine Dauer von 120 bis 180 Tagen zu schützen, wobei der Bolus kontinuierlich in den Verdauungstrakt der Wiederkäuer 0,04 mg/Tier/Tag bis 15,63 mg/Tier/Tag von LL-F28249α oder 23-(O-methyloxim)LL-F28249α abgibt.

5. Bolus nach Anspruch 4, worin der Bolus in den Verdauungstrakt der Wiederkäuer 0,2 mg/Tier/Tag bis 10,4 mg/Tier/Tag von LL-F28249α oder 23-(O-methyloxim)LL-F28249α abgibt.

6. Bolus nach Anspruch 1, zum Verhindern oder Hemmen von Nematoden- und Zeckenbefall von Rindvieh für bis zu 167 Tage, umfassend von 75 mg bis 1875 mg LL-F28249α oder 23-(O-methyloxim)-LL-F28249α, dispergiert in einer Mischung, umfassend 14,00 Gew.-% bis 16 Gew.-% Glycerinmonostearat, 3,00 Gew.-% bis 5,0 Gew.-% Carnaubawachs und 70,00 Gew.-% bis 80,5 Gew.-% feinteiliges Bariumsulfat oder Baryt.

7. Bolus nach Anspruch 6, worin der Bolus in den Verdauungstrakt der Wiederkäuer von 0,001 mg/kg Gewicht des Tierkörpers pro Tag bis 0,075 mg/kg Gewicht des Tierkörpers pro Tag von LL-F28249α oder 23-(O-methyloxim)LL-F28249α abgibt.

8. Bolus nach Anspruch 1 zur systemischen Bekämpfung des Befalls von Wiederkäuern durch ektoparasitäre Insekten, worin der Bolus in den Pansen des behandelten Tieres eine systemisch wirksame Menge von LL-F28249α oder 23-(O-methyloxim)LL-F28249α oder eines Derivates davon abgibt.

9. Bolus nach Anspruch 8, umfassend etwa 375 mg bis 1875 mg von LL-F28249α oder 23-(O-methyloxim)LL-F28249α, 14 Gew.-% bis 16 Gew.-% Glycerinmonostearat, 3 Gew.-% bis 5 Gew.-% Carnaubawachs und 70 Gew.-% bis 80 Gew.-% Baryt.

10. Bolus zum Verringern oder Verhindern der Verseuchung des Futters von Wiederkäuern durch die infektiösen Stadien von Nematoden, endoparasitären Insekten, ektoparasitären Insekten und Acarida, die die genannten Tiere befallen, umfassend 0,3 Gew.-% bis 10 Gew.-% von LL-F28249α oder 23-(O-methyloxim)LL-F28249α, 10 Gew.-% bis 20 Gew.-% Glycerinmonostearat, 3,0 Gew.-% bis 10,0 Gew.-% Carnaubawachs und 70,0 Gew.-% bis 85 Gew.-% feinteiliges Bariumsulfat oder Baryt, wobei der Bolus die Endo- und Ektoparasiten tötet und/oder ihren Fortpflanzungszyklus verhindert, so daß die Eier der Parasiten aus den Exkrementen der behandelten Tiere beseitigt werden.

## Revendications

1. Bol retard pour administration orale à des ruminants, efficace pour protéger lesdits ruminants pendant une période prolongée contre l'infestation par les adultes et les larves d'helminthes, d'insectes endoparasitaires, d'insectes ectoparasitaires et d'acariens, comprenant 0,3% à 10% en poids de LL-F28249α, de 23-(O-méthyloxime)LL-F28249α ou d'un de leurs dérivés; 10% à 20% en poids de monostéarate de glycérol; 3,0% à 10% en poids de cire de carnauba et 70,0% à 85% en poids de sulfate de baryum ou de fines de baryte.

2. Bol retard selon la revendication 1, comprenant 1,0% à 8,5% en poids dudit 23-(O-méthyloxime)LL-F28249α ou de LL-F28249α, 14% à 16% en poids de monostéarate de glycérol, 3,0% à 5,0% en poids de cire de carnauba et 70% à 80,5% en poids de baryte.

3. Bol retard selon la revendication 1, efficace pour combattre ou empêcher les infestations par les helminthes de ruminants, comprenant 0,3% à 1,0% en poids de LL-F28249α ou de 23-(O-méthyloxime)LL-F28249α, 14% à 16% en poids de monostéarate de glycérol, 3,0% à 5,0% en poids de cire de carnauba et 78,01% à 82,7% en poids de sulfate de baryum ou de baryte.

4. Bol retard selon la revendication 1, efficace pour traiter des ruminants pour les protéger contre une infestation par les helminthes, les insectes endoparasitaires, les insectes ectoparasitaires et les acariens, pendant de 120 à 180 jours, dans lequel ledit bol libère en continu, dans l'appareil digestif desdits ruminants, 0,04 mg/tête/jour à 15,63 mg/tête/jour de LL-F28249α ou de 23-(O-méthyloxime)LL-F28249α.

5. Bol selon la revendication 4, dans lequel ledit bol libère , dans l'appareil digestif desdits ruminants, 0,2 mg/tête/jour à 10,4 mg/tête/jour de LL-F28249α ou de 23-(O-méthyloxime)LL-F28249α.

6. Bol selon la revendication 1, pour empêcher ou inhiber l'infestation par les nématodes et les tiques de bovins pendant jusqu'à 167 jours, comprenant de 75 mg à 1875 mg de LL-F28249α ou de 23-(O-méthyloxime)LL-F28249α, dispersé dans un mélange comprenant 14,00% à 16% en poids de monostéarate de glycérol, 3,00% à 5,0% en poids de cire de carnauba et 70,00% à 80,5% en poids de sulfate de baryum ou de fines de baryte.

7. Bol selon la revendication 6, dans lequel ledit bol libère dans l'appareil digestif desdits ruminants, 0,001 mg/kg de poids corporel de l'animal et par jour à 0,075 mg/kg de poids corporel de l'animal et par jour de LL-F28249α ou de 23-(O-méthyloxime)LL-F28249α.

8. Bol selon la revendication 1, servant à la lutte systémique contre les infestations d'insectes ectoparasitaires de ruminants, ledit bol libérant dans le rumen de l'animal traité une quantité efficace sur le plan systémique de LL-F28249α, de 23-(O-méthyloxime)LL-F28249α ou d'un de leurs dérivés.

9. Bol selon la revendication 8, comprenant environ 375 mg à 1875 mg de LL-F28249α ou de 23-(O-méthyloxime)LL-F28249α, 14% à 16% en poids de monostéarate de glycérol, 3% à 5% en poids de cire de carnauba et 70% à 80% en poids de baryte.

10. Bol servant à réduire ou à empêcher la contamination de pâturages de ruminants par des stades infectieux de nématodes, d'insectes endoparasitaires, d'insectes ectoparasitaires et d'acariens qui infestent lesdits animaux, comprenant de 0,3% à 10% en poids de LL-F28249α ou de 23-(O-méthyloxime)LL-F28249α; de 10% à 20% en poids de monostéarate de glycérol; de 3,0% à 10% en poids de cire de carnauba et de 70,0% à 85% en poids de sulfate de baryum ou de fines de baryte, dans lequel ledit bol tue lesdits endo- et ectoparasites et/ou inhibe leur cycle de reproduction, éliminant ainsi les oeufs desdits parasites des selles des animaux traités.